Europäisches Patentamt

(19) European Patent Office

Office européen des brevets

(11) Publication number: **0 358 463**
A1

## (12) EUROPEAN PATENT APPLICATION

(21) Application number: **89308989.6**

(22) Date of filing: **05.09.89**

(51) Int. Cl.5: **A 61 K 37/24**
C 07 K 3/20, C 12 P 21/02

(30) Priority: **07.09.88 ZA 886645**

(43) Date of publication of application:
**14.03.90 Bulletin 90/11**

(84) Designated Contracting States:
**AT BE CH DE ES FR GB GR IT LI LU NL SE**

(71) Applicant: **BIOCLONES (PROPRIETARY) LIMITED**
**Grayston Medical Mews Grayston Drive**
**Sandton Transvaal (ZA)**

(72) Inventor: **Merrifield, Edwin Howard**
**8 Bramble Way**
**Oakridge Cape Province (ZA)**

(74) Representative: **Hildyard, Edward Martin et al**
**Frank B. Dehn & Co. European Patent Attorneys Imperial**
**House 15-19 Kingsway**
**London WC2B 6UZ (GB)**

(54) Purification of erythropoietin.

㉗ A method for the purification of a human erythropoietin (EPO) glycoprotein which is the product of procaryotic or eucaryotic host expression of an exogenous DNA sequence from a medium containing it includes the steps of contacting the medium, which is preferably a culture supernatant, with a support suitable for use in chromatography to which is attached a lectin, preferably wheatgerm lectin, preferentially to bind the human EPO glycoprotein and eluting the human EPO glycoprotein from the support with a suitable eluent.

EP 0 358 463 A1

Description

# PURIFICATION OF ERYTHROPOIETIN

This invention relates to a method for the purification of a human erythropoietin (EPO) glycoprotein which is the product of procaryotic or eucaryotic host expression of an exogenous DNA sequence, from a medium containing it, and to the human EPO glycoprotein so obtained.

EPO is an acidic glycoprotein with a molecular weight of approximately 34 000 daltons. The polypeptide chain consists of 166 amino acids with a leader chain of 27 amino acids and a sugar content of 24-31%. EPO is the primary regulator of red blood cell formation in mammals. EPO increases red cell production by stimulating the conversion of primitive precursor cells in the bone marrow into proerythroblasts which mature, synthesize haemoglobin and are released into the circulation as red blood cells. EPO is produced by the kidney.

Prior to the advent of recombinant DNA technology, small quantities of EPO were obtained by purification from the plasma of anaemic sheep and human EPO from urine of patients with aplastic anaemia. One method for the purification of human EPO is detailed in an article entitled "Purification of Human Erythropoietin", The Journal of Biological Chemistry, Vol 252, No 15, pages 5558 to 5564, 1977, by T Miyake, C Kung and E Goldwasser. According to this article, human EPO derived from the urine of patients with aplastic anaemia was purified to apparent homogeneity. The seven-step procedure, which included ion-exchange chromatography, ethanol precipitation, gel filtration, and adsorption chromatography, yielded a preparation with a potency of 70400 units/mg of protein in 21% yield. This represents a purification factor of 930. The purified hormone had a single electrophoretic component in polyacrylamide gels at pH 9, in the presence of sodium dodecyl sulphate at pH 7, and in the presence of Triton X-100 at pH 6. Two fractions of the same potency and molecular size, by sodium dodecyl sulphate gel electrophoresis, but differing slightly in mobility at pH 9, were obtained at the last step of fractionation.

Subsequent work on human erythropoietin is reported in an article entitled "The Role of Carbohydrate in Erythropoietin Action", Endocrinology, Vol 116, No 6, pages 2293 to 2299, 1985, by M S Dordal, F F Wang and E Goldwasser. This article deals with the carbohydrate compositions of the two forms of human EPO resulting from the procedure detailed in the article by Miyake et al. The carbohydrate compositions of the two fractions designated α-EPO and β-EPO as determined by Dordal et al are set out in the following table.

TABLE 1

| Carbohydrate composition of EPO | | |
|---|---|---|
| Sugar (ng/µg) | | |
| α-EPO | β-EPO | P |
| Fucose 18 ± 3.5 | 16 ± 0.4 | NSD |
| Galactose 56 ± 6.4 | 48 ± 0.7 | NSD |
| Mannose 41 ± 6.0 | 34 ± 4.2 | NSD |
| N-Ace-tylgluco-samine 69 ± 0.9 | 48 ± 3.2 | P < 0.001 |
| N-Ace-tylneu-raminic acid 130 ± 17.7 | 95 ± 7.6 | P < 0.05 |
| Total sugar 313 ± 25.8 | 241 ± 4.9 | P < 0.01 |
| Values are means ± SD. NSD, No significant difference | | |

This article illustrates that EPO derived from human urine is heterogeneous with respect to its carbohydrate composition.

In a note in Experentia, 29, page 758, (1973),Sieber, Iscove and Winterhalter reported that they had applied Concanavalin-A-Sepharose to the further purification of a preparation of human urinary EPO already selected for glycoprotein content by benzoic acid adsorption, and for uniformity of size. Material non-adherent to the Con-A-Sepharose column contained all the initial EPO activity. They concluded that affinity chromatography using lectins can allow selective elimination from EPO preparations of contaminating glycoproteins of similar size, the contaminating glycoproteins being adhered to the lectins with the EPO not adhering thereto.

In a later article entitled "Chromatography of Human Urinary Erythropoietin and Granulocyte Colony-Stimulating Factor on Insolublized Phytohaemagglutinin", Biochimica et Biophysica Acta, 496(1977) 146-154, Sieber reported that human urinary EPO was adsorbed to phytohaemagglutinin, the lectin from the red kidney bean Phaseolus vulgaris, coupled to agarose or porous glass and quantitatively eluted by a saturated solution of magnesium chloride to obtain a partial purification thereof. The EPO used by Sieber was initially extracted from urine by adsorption to benzoic acid, further purification by ion-exchange chromatography, and in certain cases by further affinity chromatography on Concanavalin-A-Sepharose, gel filtration and chromatography on calcium phosphate. When a DEAE-cellulose-purified sample of human urinary EPO was chromatographed on pytohaemagglutinin-Sepharose virtually all the activity was bound. Almost quantitative elution was achieved with a saturated solution of $MgCl_2$ yielding material with 11,8 times increased specific activity. Further, when a sample of human urinary EPO which had already been purified by adsorption onto benzoic acid and chromatagraphy on DEAE cellulose. Concanavalin-A-Sepharose, Sephadex G-100 and calcium phosphate was chromatographed on phytohaemagglutinin-Sepharose a further 6,7-fold increase in specific activity was achieved. This material, when subjected to electrophoresis revealed three broad bands, one sharp band and various very faint bands. Thus, the purification method did not lead to a homogeneous preparation containing EPO.

In an article entitled "Erythropoietin: Isolation by affinity chromatography with lectin-agarose derivatives", Proc. Natl.Acad.Sci. USA, vol 74, No 10, pp 4633-4635, October 1977, Spivak, Small and Hollenberg, reported that affinity chromatography using agarose-bound lectins was used to isolate EPO from crude preparations of sheep plasma and human urinary EPO. Six lectins (wheatgerm agglutinin, phytohaemagglutinin, Ricinus communis 120, soybean agglutinin, concanavalin A, and limulin) were studied. Only wheatgerm agglutinin-agarose and phytohaemagglutinin-agarose derivatives had significant affinity for erythropoietin. For human urinary EPO, using a wheatgerm agglutinin-agarose column and eluting with the appropriate lectin-specific sugar such as N-acetylglucosamine, the EPO could be separated from over 95% of the initial starting protein, resulting in an 8-fold purification and a recovery of 40%. According to the authors, the specific activity of the EPO recovered and the electrophoretic analysis of protein from the sugar eluates showed that the material purified by affinity chromatography, while comprising markedly fewer constituents than the starting material, still contained a number of proteins in addition to EPO.

In a further article entitled "Use of Immobilized Lectins and Other Ligands for the Partial Purification of Erythropoietin", Blood, Vol 52, No 6 (December), 1978, Spivak, Small, Shaper and Hollenberg reported on the ability of a variety of affinity adsorbents to isolate EPO from contaminating proteins in crude preparations of the hormone. They concluded that while affinity chromatography can be used to obtain a partial purification of EPO, it is necessary to rely on other separation techniques in addition to affinity chromatography in order to obtain EPO purified to homogeneity. For human urinary EPO, they obtained a 2-fold purification and an 80% recovery with chromatography on agarose-bound Con-A; with agarose-bound WGA or PHA a 2 to 8-fold purification was achieved depending on the agent chosen to release the hormone from the affinity adsorbent. Their data indicated that the disaccharide N,N-diacetylchitobiose is more effective than the monosaccharide N-acetylglucosamine in eluting EPO from agarose-WGA while guanidine hydrochloride is an effective eluting agent when agarose-PHA is employed as the ligand.

In an article entitled "A New Preparative Method for Isolation of Human Erythropoietin with Hydrophobic Interaction Chromatography", Blood, Vol 56, No 4 (October), 1980, page 620, Lee-Huang reported on a new preparative method for isolation of human urinary EPO using hydrophobic interaction chromatography on Phenyl-Sepharose CL4B. In this article, Lee-Huang states the affinity chromatography on Con-A-Sepharose 4B followed by WG-Sepharose 6MB or PHA(E)-Sepharose 4B served equally well as initial steps in processing crude urine concentrates. However, the urinary pigments and other impurities bind irreversibly to these lectins and full regeneration of their capacities has not been possible. The cost of these colmns and their maintenance is exceptionally high. In addition, the eluents N-acetylglucosamine or N,N-diacetylchitobiose, are much more expensive than NaOH and ethylene glycol. The operational expenses are too great to justify the routine use of these lectins for preparative processing of crude urine samples. However, the incorporation of these techniques into the purification procedure at a later stage, after the initial hydrophobic interaction chromatography, proved to be valuable. The majority of urinary pigments were removed by Phenyl-Sepharose CL4B, and full regeneration of these lectins can thus be achieved.

In an article entitled "Purification of Human Erythropoietin to Homogeneity by a Rapid Five-Step Procedure" Blood, Vol 67, No 1 (January), 1986, pages 71 to 79, Krystal, Pankratz, Farber and Smart reported that human urinary EPO has been purified using a five-step procedure to yield preparations with potencies of 80 000 U/mg in 25% yield. The five-steps involve (1) affinity chromatography on CM Affi-Gel Blue, (2) chromatofocusing, (3) wheatgerm lectin (or hydroxyl-apatite) chromatography, (4) reverse-phase high-performance liquid chromatography (HPLC) using a phenyl column, and (5) preparative sodium dodecyl sulfate-polyacrylamide gel electrophoresis (SDS-PAGE). It is to be noted that the authors of this article point out that certain EPO preparations from the chromatofocusing step were not well suited to wheatgerm fractionation, i.e. most of the EPO activity does not bind to the lectin. In these cases, the authors used hydroxyl-apatite chromatography.

To summarise, although several articles teach the partial purification of EPO from human urinary samples using lectins, it has been concluded that this process is not effective as a single step process for the purification of the EPO and further, only a maximum of a 12-fold increase in specific activity can be obtained using this process.

With the advent of recombinant DNA technology, glycoproteins have been produced possessing part or all of the primary structural conformation and one or more of the biological properties of the human EPO, the glycoproteins being a product of procaryotic or eucaryotic host expression of an exogenous DNA sequence.

For example, genomic DNA, cDNA and manufactured DNA sequences coding for part or all of the sequence of amino acid residues of EPO or for analogs thereof have been incorporated into autonomously replicating plasmid or viral vectors employed to transform or transfect suitable procaryotic or eucaryotic host cells such as bacteria, yeast or vertebrate cells in culture. The culturing of such cells results in expression of the gene or genes coding for EPO, the production of polypeptides possessing part or all of the primary structural conformation and one or more of the biological properties of the human EPO, and then glycosylation of such polypeptides in the cell culture to produce the EPO glycoproteins. Upon isolation from culture media or cellular lysates or fragments, products of expression of the DNA sequence display, for example, the immunological properties and in vitro and in vivo biological activities of EPO of human or monkey species origin. These genetically engineered EPO glycoproteins have required the development of new methods for their purification.

United States Patent No 4,677,195 to Hewick and Seehra discloses a method for purifying human EPO (which may be that from the blood or urine of patients suffering from aplastic anaemia or that from expression of genetically engineered vectors introduced into organisms for production of EPO by cell culture and fermentation processes), which comprises treating partially purified EPO having a specific activity of at least about 80000 IU per absorbance unit at 280 nanometers by reverse-phase high-performance liquid chromatography and eluting therefrom homogeneous human EPO which moves as a single peak on reverse-phase high-performance liquid chromatography and is characterised by a specific activity of at least 160000 IU per absorbance unit at 280 nanometers. This purification process, however, has the disadvantages that it is only able to deal with small quantities of medium containing the human EPO, and is expensive.

There is thus a need for an improved method for the purification of a human EPO glycoprotein which is the product of procaryotic or eucaryotic host expression of an exogenous DNA sequence.

## SUMMARY OF THE INVENTION

According to the invention there is provided a method for the purification of a human erythropoietin (EPO) glycoprotein which is the product of procaryotic or eucaryotic host expression of an exogenous DNA sequence from a medium containing it, which includes the steps of:

(a) contacting the medium with a support suitable for use in chromatography to which is attached a lectin, preferentially to bind the human EPO glycoprotein: and

(b) eluting the human EPO glycoprotein from the support with a suitable eluent.

The method of the invention provides an increase in specific activity of the EPO from the medium prior to step (a) to the eluate after step (b) of at least 50-fold, more preferably at least 80-fold.

The method of the invention preferably includes a third step, step (c) which comprises separating the human EPO glycoprotein from the eluent, for example, by chromatoaraphy.

In step (b), the suitable eluent is preferably N-acetylglucosamine.

The medium to be purifed may be a culture supernatant containing one or more human EPO glycoproteins which has not been subjected to any prior purification step.

Alternatively, the medium to be purified may be an eluate from a pre-treatment step which step comprises contacting a culture supernatant containing one or more human EPO glycoproteins with an affinity chromatography support preferentially to bind the human EPO glycoprotein or glycoproteins and then eluting the human EPO glycoprotein or glycoproteins from the support with a suitable eluent to produce the eluate.

Preferably the human EPO has been produced by a mammalian cell line, more preferably a baby hamster kidney cell line according to the method of J S Powell, K L Berkner, R V Lebo, and J W Adamson, Proc. Natl. Acad. Sci. Vol 83, pages 6465 to 6469, September 1986.

According to another aspect of the invention, there is provided a product of the method of the invention.

It is believed that the method of the invention is selective for a particular human EPO glycoprotein or class of human EPO glycoproteins over other human EPO glycoproteins which may be contained in the medium to be purified so that the product obtained is substantially homogeneous.

According to a further aspect of the invention, there is provided the product of the method of the invention, for use in the treatment of anaemia, including anaemia found in patients on renal dialysis for kidney failure, anaemia associated with cancer, aplastic anaemias, anaemia due to blood loss, anaemia associated with chronic renal disease, and to increase red cell mass prior to blood donation.

According to a yet further aspect of the invention, there is provided a pharmaceutical composition containing the product of the method of the invention.

## DESCRIPTION OF EMBODIMENTS

The first aspect of the invention is a method for the purification of a human EPO glycoprotein, which is the product of procaryotic or eucaryotic host expression of an exogenous DNA sequence from a medium containing it.

The first step of the method of the invention is to contact the medium with a support suitable for use in chromatography to which is attached a lectin, preferentially to bind the human EPO glycoprotein.

The support may be any suitable support, which is capable of having a lectin attached thereto without altering the activity of the lectin. An example of a suitable support is Sepharose.

The lectin may be any suitable lectin. The lectin must be able to recognise N-acetylglucosamine and/or sialic acid (also known as N-acetylneuraminic acid) residues. For example, the lectin may be a potato lectin or a

4

wheatgerm lectin. The preferred lectin is a wheatgerm lectin.

Generally, the support with the lectin attached to thereto will be provided in a suitable column so that the medium containing the human EPO glycoprotein can be passed through the column.

It is to be noted that the method of the invention may be carried out without any initial purification of the medium containing the human EPO glycoprotein. Thus, the medium may be, for example, a culture supernatant containing one or more human EPO glycoproteins which has not been subjected to any prior purification steps.

Alternatively, the medium to be purified may be an eluate from a pre-treatment step, which step comprises contacting a culture supernatant containing one or more human EPO glycoproteins with an affinity chromatography support preferentially to bind the human EPO glycoprotein or glycoproteins and then eluting the human EPO glycoprotein or glycoproteins from the support with a suitable eluent to produce the eluate. The support may be for example a Sepharose such as Blue Sepharose and the eluent may be a sodium chloride solution in a suitable buffer such as sodium phosphate.

In the second step of the method of the invention, the human EPO glycoprotein bound to the support is eluted therefrom with a suitable eluent. The preferred eluent is N-acetylglucosamine.

The third optional step of the method of the invention, is to separate the human EPO glycoprotein from the eluent. The preferred method to separate the human EPO glycoprotein from the eluent and from other minor impurities, is by chromatography, for example, on a DEAE-Sepharose column.

The starting medium may be a culture supernatant or may be produced from a culture supernatant which may contain one or more human EPO glycoproteins. Preferably, the culture supernatant is that produced by a mammalian cell line, preferably that produced by a baby hamster kidney cell line.

It has been found that the method of the invention selects for a particular human EPO glycoprotein or class of human EPO glycoproteins over other human EPO glycoproteins which may be found in the medium.

Using the method of the invention it has been possible to produce a recombinant human EPO glycoprotein having a specific activity of 200 000 U $\pm$ 10 000 U per mg of protein, determined using a standard of known concentration, viz. 50 $\mu$g of recombinant human EPO per ml, from a medium having a specific activity of 2 000 U per mg of protein, i.e. a 100-fold increase in specific activity. This is a significant improvement in specific activity over what has been achieved utilizing previously known processes.

The second aspect of the invention is the product of the method of the invention which is believed to be purified, homogenous, human EPO, or a class of human EPOs.

The product of the method of the invention may be used in the treatment of various types of anaemia, and for this purpose may be formulated into pharmaceutical compositions suitable for such use.

Examples of the method of the invention will now be given.

## EXAMPLE 1

This example relates to the purification of human recombinant EPO directly from cell culture supernatant fluid (CSF) using a two-step procedure.

Crude cell culture supernatant fluid is buffered with sodium phosphate (pH 7,0 - 7,5, 0,01 M final concentration) and applied directly to a column of wheatgerm lectin-Sepharose. This column is washed with 5 volumes of 0,01 M sodium phosphate buffer to remove unbound material and then eluted with 0,2 M N-acetylglucosamine in 0,01 M sodium phosphate buffer to effect the release of EPO. The material eluting from this column is pumped directly onto a column of DEAE-Sepharose previously equilibrated with phosphate buffer (0,01 M, pH 7,0). The column is washed with 10 volumes of buffer to ensure the complete removal of N-acetylglucosamine and then eluted with phosphate buffered saline (PBS) to release the bound EPO.

Purified EPO migrated as a single band on SDS-PAGE (15% gel used) when visualized with both Coomassie Blue and silver staining procedures.

Analysis by reverse-phase HPLC indicated that purified EPO eluted as a single peak with a retention time of 36-37 minutes under the following conditions.

Column. Beckman Ultrapore RPSC C3 4,6 mm i.d. x 75 cm
Solvent A. 0,05% Trifluoroacetic acid in water
Solvent B. 0,05% Trifluoroacetic acid in acetonitrile
Gradient. 0% B to 70% at 1% per minute
Detection. UV absorbance at 206 nm.

Material prepared according to this procedure has been assayed for biological activity according to the $^3$H-thymidine assay of Krystal, G., Exp. Hematol. 11 (1983) 649, and exhibits a specific activity of not less than 200 000 units per milligram of protein.

The results of six runs of the above example are set out below in Table 2.

TABLE 2

| Run No | CSF (ml) | EPO (µg) | WGL (µg) | DEAE (µg) | % YIELD ON WGL |
|--------|----------|----------|----------|-----------|----------------|
| 1 | 20 | 250 | 154 | 133 | 61,6 |
| 2 | 20 | 250 | 156 | 137 | 62,4 |
| 3 | 20 | 250 | 156 | 148 | 62,4 |
| 4 | 20 | 250 | 155 | 122 | 62,0 |
| 5 | 20 | 250 | 153 | 134 | 61,2 |
| 6 | 20 | 250 | 151 | 127 | 60,4 |

CSF = Culture supernatant fluid

WGL = Amount of EPO recovered on wheatgerm lectin support

DEAE = Amount of EPO recovered after removal of eluent on DEAE-Sepharose

The results show a consistent percentage yield on a wheatgerm lectin support of at least 61-62%.

The remaining EPO can be recovered by successively eluting the column with 0,1 M N-acetylneuraminic acid and finally with a chaotropic agent such as 4 M guanidinium hydrochloride.

**EXAMPLE 2**

As an alternative to purifying human recombinant EPO directly from cell culture supernatant fluid (CSF), the CSF may first be subjected to the following pre-treatment step.

Crude CSF (400 ml/column) is pumped onto 4 columns (10 ml gel capacity) containing Blue-Sepharose CL-6B (Pharmacia) using a multi-channel peristaltic pump set to deliver 1,5 ml/min. After the crude solution has been loaded, the columns are washed with 0,01 M sodium phosphate buffer pH 7,0 (20 ml/column) at the same flow-rate.

The bound material containing predominantly the EPO is then eluted from the column with 1 M sodium chloride in 0,01 M sodium phosphate buffer pH 7,0 (20 ml/column). At this stage the preparation is 65% pure as assessed by reverse-phase HPLC and the recovery from Blue-Sepharose is 80%.

Blue-Sepharose is an affinity matrix of fairly broad specificity used primarily for the removal of serum albumin. The matrix contains 1,7 -2,3 µmoles Cibacron Blue ® F3G-A per ml of gel.

The preparation produced by this pre-treatment step is then subjected to the two-step procedure set out in Example 1.

The method of the invention permits the recovery of a significant amount of human EPO glycoprotein, from a medium containing it in an easy and economical manner. Further, the method of the invention has the advantage that it is capable of dealing with large quantities of medium containing the human EPO to be purified.

In the light of prior art, as set out in the background to the invention, it is unexpected that the method of the invention can provide a 100-fold increase in specific activity between the starting medium and the eluate given that the prior art teaches a maximum 12-fold increase in specific activity. Further, it is unexpected that the method of the invention can be utilized without prior purification of the medium containing the EPO. Finally, it is unexpected that the method of the invention produces a product which is substantially homogeneous.

**Claims**

1. A method for the purification of a human erythropoietin (EPO) glycoprotein which is the product of procaryotic or eucaryotic host expression of an exogenous DNA sequence from a medium containing it, including the steps of:

(a) contacting the medium with a support suitable for use in chromatography to which is attached lectin, prefer-entially to bind the human EPO glycoprotein; and

(b) eluting the human EPO glycoprotein from the support with a suitable eluent.

2. A method according to claim 1 which includes a third step:

(c) separating the human EPO glycoprotein from the eluent.

3. A method according to claim 2 wherein in step (c) the human EPO glycoprotein is separated from the eluent by chromatography.

4. A method according to any of the preceding claims wherein there is an increase in the specific activity of the EPO from the medium prior to step (a) relative to the EPO in the eluate after step (b) of at least 50-fold.

5. A method according to claim 4 wherein said increase is at least 80-fold.

6. A method according to any of the preceding claims wherein in step (a) the support is Sepharose.

7. A method according to any of the preceding claims wherein in step (a) the lectin is potato lectin.

8. A method according to any of claims 1 to 6 wherein in step (a) the lectin is wheatgerm lectin.

9. A method according to any of the preceding claims wherein in step (b) the eluent is N-acetylglucosamine.

10. A method according to any of the preceding claims wherein the medium is a culture supernatant containing one or more human EPO glycoproteins which has not been subjected to any prior purification step.

11. A method according to claim 10 wherein the culture supernatant is that produced by a mammalian cell line.

12. A method according to claim 11 wherein the culture supernatant is that produced by a baby hamster kidney cell line.

13. A method according to any of claims 1 to 9 wherein the medium is an eluate from a pre-treatment step comprising contacting a culture supernatant containing one or more human EPO glycoproteins with an affinity chromatography support preferentially to bind the human EPO glycoprotein or glycoproteins and eluting the human EPO glycoprotein or glycoproteins from the support with a suitable eluent to produce the eluate.

14. A method according to claim 13 wherein the culture supernatant is that produced by a mammalian cell line.

15. A method according to claim 14 wherein the culture supernatant is that produced by a baby hamster kidney cell line.

16. A purified human erythropoietin (EPO) glycoprotein which is the product of procaryotic or eucaryotic host expression of an exogenous DNA sequence, when purified by a method according to any one of claims 1 to 15 from a medium containing the human EPO glycoprotein, the purified human EPO glycoprotein having a specific activity of about 200,000 U per mg of protein.

17. The purified human EPO glycoprotein of claim 16 for use in the treatment of anaemia, including anaemia found in patients on renal dialysis for kidney failure, anaemia associated with cancer, aplastic anaemias, anaemia due to blood loss, anaemia associated with chronic renal disease, and to increase red cell mass prior to blood donation.

18. A pharmaceutical composition containing as the active ingredient the purified human EPO glycoprotein of claim 16.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | HAEMATOLOGICA, vol. 63, no. 4, August 1978, pages 426-430; Z. RUDZKI et al.: "The use of wheat germ lectin in the purification of erythropoietin" * Whole article * | 1-18 | A 61 K 37/24 C 07 K 3/20 C 12 P 21/02 |
| X | EP-A-0 232 034 (SUMITOMO PHARMACEUTICALS CO. LTD) * Page 15, lines 1-27 * | 1-18 | |

TECHNICAL FIELDS SEARCHED (Int. Cl.5)

A 61 K
C 07 K
C 12 P

The present search report has been drawn up for all claims

| Place of search | Date of completion of the search | Examiner |
|---|---|---|
| THE HAGUE | 31-10-1989 | TURMO Y BLANCO C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)